(19) Europäisches Patentamt
European Patent Office
Office européen des brevets

(11) Publication number: 0 074 138
A1

# (12) EUROPEAN PATENT APPLICATION

(21) Application number: 82201051.8

(22) Date of filing: 23.08.82

(51) Int. Cl.³: C 07 D 493/04
A 61 K 31/35
//(C07D493/04, 311/00, 307/00)

(30) Priority: 31.08.81 DE 3134364

(43) Date of publication of application:
16.03.83 Bulletin 83/11

(84) Designated Contracting States:
AT BE CH DE FR GB IT LI LU NL SE

(71) Applicant: Chem. Pharmaz. Fabrik Dr. Hermann Thiemann GmbH
Kirchstrasse 12 - 16
D-4670 Luenen/Westf.(DE)

(72) Inventor: Eiden, Fritz
Sophienstrasse 10
D-8000 München(DE)

(72) Inventor: Schünemann, Jürgen
Adalbertstrasse 110
D-8000 München(DE)

(72) Inventor: Mayer, Dieter
Beethovenstrasse 1
D-4712 Werne(DE)

(74) Representative: Hermans, Franciscus G.M. et al,
Postbus 20
NL-5340 BH Oss(NL)

## (54) Furano-chromone derivatives.

(57) The invention relates to new furano-chromone derivatives of the general formula:

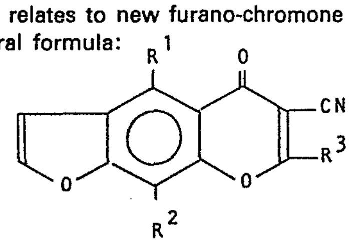

wherein

$R^1$ represents ($C_1$-$C_6$) alkoxy, hydroxyl or ω-[di-($C_1$-$C_6$) alkyl-amino]-($C_1$-$C_6$) alkoxy

$R^2$ represents hydrogen or ($C_1$-$C_6$) alkoxy and

$R^3$ represents hydrogen, ($C_1$-$C_6$) alkyl, phenyl or $R^4$-phenyl wherein

$R^4$ is halogen, ($C_1$-$C_6$) alkoxy or trifluoromethyl, which can be used as medicaments and have, above all, analgesic, anti-inflammatory, anti-thrombotic and blood-platelet aggregation-inhibiting properties.

EP 0 074 138 A1

Croydon Printing Company Ltd.

# FURANO-CHROMONE DERIVATIVES

The invention relates to new furano-chromone derivatives, to processes for their preparation and to pharmaceutical preparations containing same.

More particularly the invention relates to furano-chromone compounds of the general formula I:

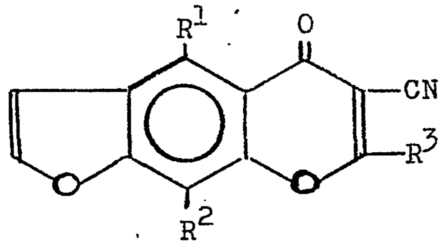

wherein $R^1$ denotes $(C_1-C_6)$ alkoxy, hydroxyl or ω- [di-$(C_1-C_6)$ alkyl - amino]-$(C_1-C_6)$ alkoxy, $R^2$ represents hydrogen or $(C_1-C_6)$ alkoxy and $R^3$ hydrogen, $(C_1-C_6)$ alkyl, phenyl or $R^4$-phenyl, wherein $R^4$ denotes halogen, $(C_1- C_6)$ alkoxy or trifluoromethyl.

Where a $C_1-C_6$-alkyl radical occurs in one of the radicals $R^1$ to $R^4$, this alkyl radical can be, for example, methyl, ethyl, n-propyl, isopropyl, n-butyl, isobutyl, t-butyl, n-pentyl, isoamyl or n-hexyl. $C_1-C_4$ radicals, in particular methyl and ethyl, are preferred.

The substituent $R^4$ can be in the o-, p-, or m-position whereby the p-position is preferred. If $R^4$ represents halogen, this is chlorine, fluorine or bromine, preferably chlorine or fluorine.

The compounds of formula I may be prepared by reacting a compound of formula II:

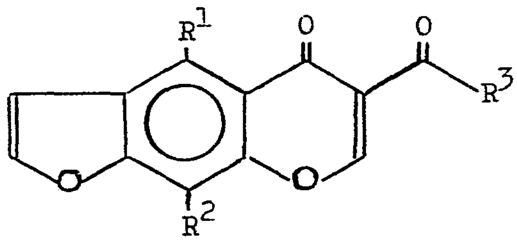

II

inwhich $R^1$, $R^2$ and $R^3$ have the meanings indicated above, with hydroxylamine or an acid addition salt thereof.

This reaction is preferably carried out at a temperature above about 40°C. The hydroxylamine is usually employed as a salt, which is dissolved e.g. in formic acid and sodium acetate or in alkalimetalhydroxyde or pyridine.

The starting compound of formula II can be prepared at various manners.
The flow sheet on the next page shows some manners for the preparation of the starting compounds of formula II.

The compounds of formula III (see flow sheet) can be obtained by hydrolysis of khellin or visnagin or of derivatives thereof. The compounds of the general formula IV are obtained from compounds of the general formula III by reaction with the appropriate acidchlorides or anhydrides in the presence of a base.

The reaction V→ II is preferably carried out at temperatures below 40°C, in particular below 30°C. The reaction is usually carried out in the presence of solvents, such as aromatic hydrocarbons or halogenohydrocarbons.

The preparation of compounds according to the

Flow sheet

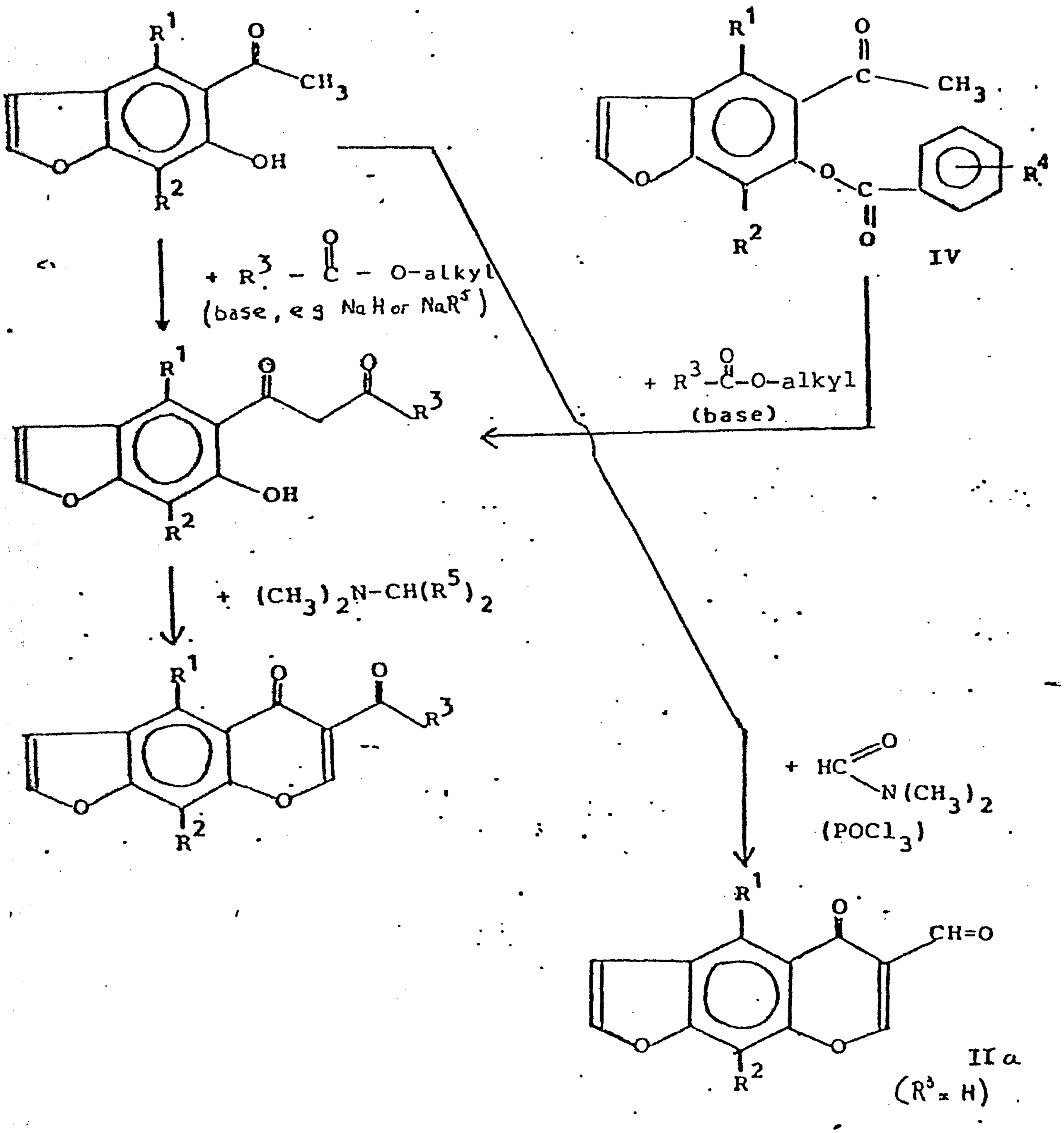


In the formulae, $R^5$ denotes methoxy, ethoxy or i-propoxy.

invention in which $R^3$ denotes hydrogen can in addition be carried out in accordance with the reaction III → IIa.

A temperature below $50^oC$ is preferably used for this reaction (III → IIa). An excess of dimethylformamide is used as the solvent. The reaction with dimethylformamide is followed by hydrolysis using e.g. a mixture of ice and water.

The compounds according to the invention have, above all, analgesic, anti-inflammatory, anti-thrombotic and blood platelet aggregation-inhibiting properties.

The compounds according to the invention can be processed to the customary liquid or solid pharmaceutical preparations, for example to dragees, tablets, pills, suppositories and solutions, also for injection. The customary excipients, carriers and diluents and the usual procedures for the preparation of these pharmaceutical preparations are used.

The usual individual dose is 2 to 20 mg/kg orally and 0.5 to 5 mg/kg intravenously, and the customary daily doses are generally three times these individual doses mentioned.

Particularly preferred compounds according to the invention are compounds of formula I, inwhich $R^1$ and $R^2$ are both alkoxy with 1-4 carbonatoms and especially methoxy, and inwhich $R^3$ preferably represents hydrogen or phenyl.

## Example 1

(3-Cyano-5,9-dimethoxy-furo-[3,2-g]-$\Delta^2$-chromen-4-one)

a. 3-Formyl-5,9-dimethoxy-furo-[3,2-g]-$\Delta^2$-chromen-4-one

122.8 g (0.8 mol) of $POCl_3$ are added dropwise to a solution of 47.23 g (0.2 mol) of khellinone in 750 ml of dimethylformamide such that the temperature

of the reaction-solution does not exceed 40$^{\circ}$C. After a further 250 ml of dimethylformamide have been added, the mixture is stirred for another hour at an internal temperature of at the most 45$^{\circ}$C.

After the reaction mixture has been cooled to room temperature, it is hydrolysed in ice/water. The yellowish precipitate is filtered off with suction and recrystallised from acetonitrile; yield (after recrystallising once): 88%; melting point 206-207$^{\circ}$C.

b. 3-Cyano-5,9-dimethoxy-furo-[3,2-]-$\Delta^2$-chromen-4-one

A solution of 18.2 g (0.26 mol) of $NH_2OH.HCl$ and 13.6 g (0.2 mol) of sodium formate in 150 ml of formic acid is added to a solution of 27.42 g (0.1 mol) of 3-formyl-5,9-dimethoxy-furo-[3,2-g]-$\Delta^2$-chromen-4-one in 200 ml of acetic acid and the mixture is stirred at 50$^{\circ}$C for 4 hours. After the mixture has been cooled to room temperature, it is poured on to ice/water and left to stand overnight. The precipitate is filtered off with suction and warmed to 70$^{\circ}$C in 1000 ml of 10% strength KOH solution ($C_2H_5OH/H_2O$ = 1:1) for 2 hours. After the mixture has been cooled to room temperature, it is acidified with dilute hydrochloric acid. The precipitate is recrystallised from acetonitrile; yield (after recrystallising twice): 54.4%; dark brown crystals of melting point 245-247$^{\circ}$C.

## Example 2

### 3-Cyano-2-phenyl-5,9-dimethoxy-furo-[3,2-g]-$\Delta^2$chromen-4-one

a. 3-Benzoyl-2-norkhellin

A solution of 0.01 mol of 5-(benzoylacetyl)-4,7-dimethoxy-6-hydroxy-benzofuran in 15 ml of absolute $CHCl_3$ is added drop wise to 0.02 mol of dimethylamino-

formamideacetal at room temperature. When the reaction has ended (about 30 minutes), the mixture is evaporated to dryness in vacuo, the residu is triturated to a paste, using silica gel and xylene/ acetone (8:2) as the eluting agent, and the paste is introduced on to a silica gel column (0.55 mm).

The column is eluted with xylene/acetone (8:2), the eluate is evaporated to dryness in vacuo and the residue is recrystallised twice from n-butanol; yielding orangecoloured crystals of melting point 178-179°C; yield: 43%.

b. 3-Cyano-2-phenyl-5,9-dimethoxy-furo-[3,2-g]-$\Delta^2$-chromen-4-one

1.05 g of the 3-benzoyl-2-norkhellin obtained are dissolved in 100 ml of 10% strength KOH, with warming, and 0.42 g of $NH_2OH.HCl$ are added . The reaction mixture is stirred for 4 hours. It is then poured into 150 ml of 10% strength HCl and the precipitate is filtered off with suction. This precipitate is heated at 90°C in 50 ml of 10% strength KOH ($EtOH:H_2O = 1:1$) for 30 minutes. After the mixture has been cooled to room temperature, it is acidified and the precipitate is recrystallised from isopropanol; yellow-orange coloured crystals of melting point 213-214°C are obtained. Yield 20%.

## Example 3

In a manner corresponding to Example 2 are prepared:

3-Cyano-2-methyl-5,9-dimethoxy-furo-[3,2-g]-$\Delta^2$-chromen-4-one ;

3-Cyano-2-phenyl-5-methoxy-furo-[3,2-g]-$\Delta^2$-chromen-4-one ;

3-Cyano-2-phenyl-5-hydroxy-furo-[3,2-g]-$\Delta^2$-chromen-4-one,
3-Cyano-2-(4-chloro) phenyl-5,9-dimethoxy-furo-[3,2-g]-$\Delta^2$-chromen-4-one.

CLAIMS

1. Furano-chromone compounds of the géneral formula

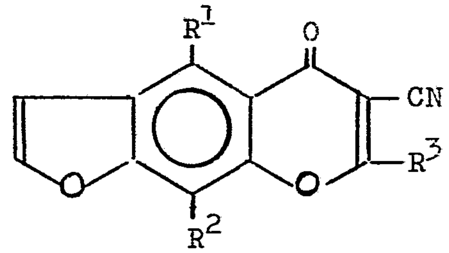

wherein $R^1$ represents $(C_1-C_6)$ alkoxy, hydroxyl or ω-[di-$(C_1-C_6)$ alkyl-amino]-$(C_1-C_6)$ alkoxy,
$R^2$ represents hydrogen or $(C_1-C_6)$ alkoxy and
$R^3$ represents hydrogen, $(C_1-C_6)$ alkyl, phenyl or $R^4$-phenyl, wherein
$R^4$ is halogen, $(C_1-C_6)$ alkoxy or trifluoromethyl.

2. Compound according to claim 1, inwhich $R^1$ and $R^2$ are both methoxy and $R^3$ is hydrogen.

3. Compound according to claim 1, inwhich $R^1$ and $R^2$ are both methoxy and $R^3$ is phenyl.

4. Process for the preparation of a compound indicated in claim 1, characterised in that the compound is prepared in a manner in actual use or described for the preparation of analogous compounds.

5. Process according to claim 4, characterised in that a compound of the general formula

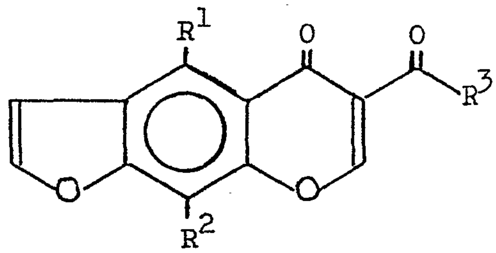

in which $R^1$, $R^2$ and $R^3$ have the meanings assigned in claim 1, is reacted with hydroxylamine or an acid addition salt thereof.

6. Pharmaceutical composition containing a compound as claimed in claim 1 as the active constituent in admixture with one or more usual pharmaceutical carriers or diluents.

European Patent Office

# EUROPEAN SEARCH REPORT

0074138
Application number

EP 82 20 1051.8

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | FR - M - 46 CAM (LABORATOIRES J. BERTHIER S.A.) * abstract * -- | 1-3,6 | C 07 D 493/04<br>A 61 K 31/35<br>//(C 07 D 493/04, 311/00, 307/00) |
| P,A | US - A - 4 313 883 (R.B. GAMMILL) * claim 1 * ---- | 1-3 | |
| | | | TECHNICAL FIELDS SEARCHED (Int.Cl. 3) |
| | | | A 61 K 31/35<br>C 07 D 493/04 |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 25-11-1982 | IDEZ |